# EUROPEAN PATENT SPECIFICATION

(11) **EP 3 171 778 B1**
(45) Date of publication and mention of the grant of the patent: **25.04.2018**
(21) Application number: 15756993.0
(22) Date of filing: 21.07.2015
(51) Int. Cl.: A61B 5/15

(54) **KIT FOR CORD BLOOD COLLECTION**
KIT ZUR ENTNAHME VON NABELSCHNURBLUT
KIT DE COLLECTE DE SANG DE CORDON OMBILICAL

(30) Priority: 21.07.2014 PL 40894314
(43) Date of publication of application: 31.05.2017
(73) Proprietor: Pomorski Uniwersytet Medyczny w Szczecinie, 70-204 Szczecin (PL)
(72) Inventor: MACHALINSKI, Boguslaw, 71-806 Szczecin (PL); BARTKOWIAK, Artur, 71-667 Szczecin (PL); RUDNICKI, Jacek, 70-776 Szczecin (PL); KOTOWSKI, Maciej, 72-003 Bezrzecze (PL)
(74) Representative: Witek, Rafal
(86) International application number: PCT/IB2015/055521
(87) International publication number: WO 2016/012944

(56) References cited:
- EP-A1- 2 459 245
- WO-A2-2005/041772
- US-A1- 2013 190 653

## Description

The object of the invention is a kit for cord blood collection. The kit enables effective blood collection even from relatively small umbilical cord and placenta. Blood collection is achieved thanks to the gravitational force coupled with suction system, in order to facilitate its collection.

Potential usage of cells isolated from cord blood and placenta for standard therapeutic purposes may be effective provided that the volume of collected blood allows for the isolation of at least 1,5 x 107 mononuclear cells for 1 kg of patient's body mass in order to safely conduct successful transplantation.

However, currently known cord blood collection techniques allow collection of not more than a half of cord blood left after the childbirth in the umbilical cord and the placenta. This fact causes substantial utility limitation of techniques currently being used, because mean volume of collected cord blood and the number of stem cells included therein is sufficient for the transplantation only in case of patient whose body mass is below 40 kg. Adults awaiting hematopoietic transplantations are therefore excluded from transplantation procedure based on one unit of collected cord blood. Then, they need transplantation of more than one unit of antigenically compatible cord blood, which makes the whole procedure more complicated and significantly more expensive, or they need transplantation of hematopoietic cells obtained from the bone marrow or mobilised peripheral blood, which in turn requires compatible family donor or finding compatible allogenic alive donor in available donor registries, which is not always possible.

At the moment, cord blood banks store blood collected by gravimetric method. Such system usually contains a bag of volume 250 mL, bags for blood collection equipped with tubes connected with a needle 16G and about 40 mL-reservoir containing anticoagulant solution. Blood collection conducted by a specialist qualified in transplantation, transfusionist, gynaecologist/obstetrician or midwife under the supervision of specialised doctor is a process, which takes from 10 to 30 minutes, wherein blood coagulation in cord begins shortly after childbirth. Due to small diameter (about 0.5 mm) of needle inserted into umbilical vein having the diameter of 2-4 mm, blood collection time is relatively long, especially with gravimetric method. This has disadvantage effect on the volume of collected blood, which directly results in the number of collected stem cells. As already mentioned, mean volume of blood collected in this method is about 70-80 mL, which is not sufficient for therapeutic applications in adults.

Nowadays, most arrangements aimed at collection of blood from the umbilical cord and placenta during childbirth is constructed based on the systems which allow for cord puncture/perforation immediately after childbirth before cutting-off the placenta and introduction of the dedicated needle connected with a tube and with a container in a shape of bag. Blood available in cord and placenta is collected to a special bag due to the gravitation that is as a result of bag placement below the level of umbilical cord of woman lying down on a bed. Bag usually contains a special anticoagulant and it is constantly kept in move during collection process, which is the reason why blood is collected uniformly and then stored in optimal conditions. Mean volume of blood collected during childbirth with the use of above method is estimated for about 70-80 mL. Although, in many cases such amount is suitable for collection of recommended number of stem cells, numerous situations are reported where collected blood volume is not sufficient. It is therefore so important to develop a method allowing for fast and efficient collection of cord blood with the use of kit enabling easy collection and effective blood storage in amounts greater than so far, which is possible to obtain with the use of gravimetric method. A device for cord blood collection in known from WO2007031999, which is equipped with apparatus for partial cord immobilisation, which is connected with disinfectant chamber, which is connected with blood reservoir by a tube. Blood flows gravitationally, there is no possibility to control blood collection rate. Anticoagulant is present in the reservoir or it is injected. From the same description an apparatus is known for cord blood collection, which is consisted of a bag - reservoir connected with three tubes ended with needles. Blood is collected gravitationally, another needle is inserted above previous insertion site when the rate drops.

From the description of US20020183679 invention a device is known having at least one collection needle connected with collection tube and with a reservoir. Collection tube is also connected with washing solution-containing reservoir by an additional tube. Originally, anticoagulant may be in the container or it may be in an additional, smaller container connected with collection tube by a means of connecting tube. Moreover, reservoir may be connected with two additional reservoirs (if blood is divided into fractions, one of the fractions is introduced into each reservoir).

In order to accelerate collection process itself, systems are used that enable usage of special devices lowering pressure inside the device/reservoir for blood accumulation relative to blood pressure in cord and/or placenta. The amount of collected blood is increased due to the replacement of standard procedure, which uses gravimetric method, by a procedure based exclusively on underpressure created by air suctioning or usage of special dehydrating agents. Such systems contain one or more needles for puncturing of cord and/or placenta, blood collection systems, such as reservoirs, bags or vacuum containers, as well as sucking devices, such as vacuum pumps, peristaltic pumps or syringes. A device for cord blood collection is known from WO2005041772, which contains reservoir with sucking element and collection tube, as well as at least one needle for blood collection. The device may include mixing element. Anticoagulants may be introduced into the reservoir by the tube or placed inside reservoir in the internal bag, which is connected in such a wasp as to enable anticoagulant pouring into the reservoir. Collection rate is regulated by valves at the collection tube. This system is intended for collection and storage of larger amounts of blood. From US5097842 a device is known for body fluids collection, which enable supply to at least two vacuum reservoirs or test tubes with the use of one needle. The needle is connected with the device for fluids separation containing valves and inlet chamber, two outlet chambers connected by middle chamber. The valves enable supplying to several containers. The device enable filling of several containers already with one injection. The use of the device is complicated and expensive. From US5059168 a device is known with a needle connected to a tube with a valve, which first inlet is connected with a syringe with a plunger, and the second inlet with separation reservoir. The syringe is placed in sealed bag. Separation container has multiple inlets into which blood reservoirs may be connected. Blood is aspired by a syringe and then blood is pressed with the use of special valve into reservoir. Blood reservoirs are closed by tourniquets. The device is very complicated and it is unsuitable for direct centrifugation following simple separation and is unsuitable for further, simple processing after centrifugation. Solutions are also known, where sucking systems supports gravimetric method. From US20130190653 the device is known with at least one collection syringe, at least one tube, at least one reservoir for gravitational collection and at least one syringe. The syringe is directly connected with the tube (this connection may be located at the tube entrance into the reservoir) and allows increasing blood collection efficacy. From EP2459245 the device is known with at least one needle, at least one collection tube, which is connected with at least one reservoir. Blood flows into the reservoir by gravity. The device has a syringe that causes under-pressure, which is at least equal to that required for an increase of the amount of collected blood and lower than that, which could damage the placenta. Anticoagulant is at the additional reservoir. The syringe is permanently connected with a bag for blood collection, which prevents usage of typical processes used in subsequent cord blood processing, i.e. centrifugation, as in this case, there is a danger and risk of rupture of such collection system.

Usage of sucking devices (alone or in combination with gravimetric method) finally enables increasing the amount of collected blood, but it requires high financial means due to complicated sucking devices or is impractical at later stages of stem cells processing and requires usage of the new containers at subsequent steps, inter alia during centrifugation. As a result of constant underpressure, possible is blood coagulation or haemolysis at the stage of collection or occurrence of complications in a form of bruises, and hence premature placenta loosening. Therefore, it is necessary to use a simple method enabling fast change of underpressure during blood collection process.

Accordingly, it seemed necessary to develop a simple system, which will make it possible to increase volume of blood collected from a cord and placenta in such way, that the number of collected stem cells is sufficient in accordance to standards required for therapeutic purposes, and at the same time, the process is safe for mother directly after childbirth, thus guaranteeing natural placental expulsion without the risk to patient's health. In accordance with the present invention, there is provided a kit for cord blood collection as defined in claim 1.

Syringes used in the kit may be operated manually or with the use of syringe pump. In the simplest embodiment, the reservoir has a form of a bag. The size of reservoir is adjusted to expected amount of blood to be collected. Preferably, the collection tube and the tube are equipped with closing elements in a form of clamps and/or closing valves. It allows for sealing of collected blood in a container and subsequent disconnection of syringes without reservoir unsealing. Closing elements allow closing anticoagulant supply during blood sucking with the use of another syringe.

Solution of the invention has the advantage in that the collection of blood by gravimetric method is supported by a simple system in a form of syringe creating underpressure in a reservoir, which may be easily disconnected from the blood reservoir. Usage of syringe, which forms the sucking system enables collection of blood contained in the cord, as well as in the placenta by one needle insertion into umbilical vein. No necessity for multiple placenta perforations is especially important, because mentioned puncture usually incurs the risk of bacterial contamination, which could eliminate collected blood from further processing. Suction system facilitates full filling and wetting of walls of blood reservoir, both with anticoagulant and, finally, with blood. Another advantage of the solution is that the first of syringes is filled with anticoagulant, which may be dosed into blood reservoir as required. Anticoagulant is injected into the reservoir before proceeding to blood collection and is possibly injected again during blood collection, when it turns out that there is more blood collected than it was expected at the beginning of the procedure, i.e. by calculating the week of pregnancy and fetus weight predicted by USG. It enables adjusting amount of anticoagulant to temporary demand, which prevents its excessive amount in the reservoir relative to collected blood, which was not possible in solutions known from the prior art. While attempting transfusion, too much anticoagulant, and usually those are citrate solutions, leads to binding of calcium ions and in consequence to the disruption of ion balance in the body of the newborn, thus creating a risk of serious complications. In all solutions to date, it is not always possible to give transfusion of whole volume of collected blood due to physiological limits (defined as volume of fluids per kg of body mass in a single administration) because of too big anticoagulant volume. Instead of the part of the blood, the newborn would receive mainly anticoagulant volume. The use of two syringes allows to keep maximum insulation of the system and to minimise the possibility of blood contamination during the collection, as well as obtaining optimal proportion between blood and anticoagulant. The use of branched tube for connecting syringes with reservoir is beneficial as it is possible to clamp one tube faster than two separate ones. Moreover, anticoagulant possibly additionally injected again has better access to the reservoir and the sucking system-forming syringe, and blood is mixing better with anticoagulant during sucking. Thanks to clamping elements, syringes and the needle may be disconnected during collection of blood to the reservoir that remains insulated. Following the disconnection of syringes, reservoir with collected blood may be stored or used for subsequent processing. The invention allows to use reservoir of a size adapted to premature neonates (smaller reservoir), and two syringes system will enable to collect bigger amount of blood with subsequently added proportional amount of anticoagulant for further processing or clinical procedures (e.g. autohaemotransfusion). It allows for administration (transfusion) of cord blood with optimal/minimal amount of anticoagulant, but not disproportionally high amount of anticoagulant and small amount of blood accumulated in reservoir's pits, as it has been up until now. Smaller size of blood reservoir facilitates its storage in tank with nitrogen vapours (possibility to use smaller frosting cassette), and hence banking of higher number of bags in the same tank in comparison to standard bags, which are larger in volume (dimensions).

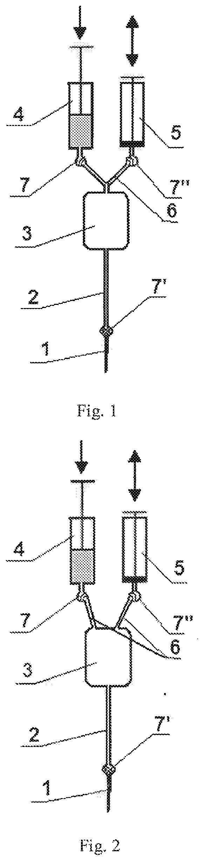

The invention is described in more details in the embodiments below and in the figure, where in Fig. 1 kit with branched tube is presented, and in Fig. 2 system with two tubes linking reservoir with syringes is presented.

### Example I

Kit for cord blood collection equipped with the needle (1) for cord blood collection connected with the collection tube (2) with the reservoir (3) in a form of elastic bag. The kit has two syringes, the first (4) and the second (5) connected with the reservoir (3) by the branched tube (6). The first syringe (4) is connected with the first branch of the tube (6), the second syringe (5) is connected with the second branch of the tube (6) and the third end of the tube (6) is connected with the reservoir (3). The first syringe (4) contains liquid anticoagulant. The collection tube (2) and the tube (6) are equipped in closing valves (7).

The valve (7) is opening on the first branch of tube (6) connected with the first syringe (4). Anticoagulant is introduced to the reservoir (3) with the use of first syringe (4) [the amount of it depends on the predicted amount of blood to be collected]. Anticoagulant fills the space within reservoir (3) and wets its walls and the tube (6). Next, the valve (7) and the valve T on the collection tube (2) is closing and the needle (1) is used to inject into umbilical vein [possibly in umbilical vein system]. The valve (7') is opening and cord blood collection into the reservoir (3) starts with gravimetric method. Once the decrease in the rate of reservoir (3) filling is observed, the valve (7") is opening on the second branch of tube (6) and with the use of the second syringe (5) [pulling the plunger of the syringe] slight underpressure is created in the reservoir (3), which is responsible to further blood collection. Once the bag is filled or after the end of blood collection, the valve (7') is closing on the collection tube (2). Blood from the second syringe (5) is injected back to the reservoir (3) and next valve (7") is closing at the second branch of tube (6). Both syringes (4), (5) and the needle (1) are disconnected. The reservoir (3) with the blood may be used directly for subsequent processing or further storage.

If during blood collection it turns out that there is more of blood than predicted in the prognosis before collection, valve (7) is opening on the first branch of the tube (6) and additional dose of anticoagulant is injected. Following administration of the amount of anticoagulant, valve (7) is closing.

### Example II

The kit made and operating analogically as in the Example I, wherein the first syringe (4) is connected with the reservoir (3) by the tube (6), and the second syringe (5) is connected by the tube (6'). Closing elements (7) are in the form of clamps.

## Claims

1. A kit for cord blood collection comprising a needle (1), a collection tube (2), a first syringe (5) and a reservoir (3), **characterized in that** at least one reservoir (3) is connected with at least one needle (1) for blood collection by the collection tube (2) and said kit comprises a second syringe (4) whereby the two syringes (4, 5) are connected with the reservoir (3) by a branched tube (6) or by separate tubes (6), wherein the first syringe (4) contains an anticoagulant, and the second syringe (5) forms a sucking system, supporting blood dripping in a gravitational manner.

2. The kit for cord blood collection according to Claim 1, **characterized in that** the collection tube (2) and the tube (6) comprise closing elements (7) in a form of clamps and/or closing valves.

## Patentansprüche

1. Kit zur Entnahme von Nabelschnurblut, aufweisend eine Nadel (1), ein Entnahmeröhrchen (2), eine erste Spritze (5) und einen Behälter (3), **dadurch gekennzeichnet, dass** zur Blutentnahme durch das Entnahmeröhrchen (2) mindestens ein Behälter (3) mit mindestens einer Nadel (1) verbunden ist und das Kit eine zweite Spritze (4) aufweist, wobei die zwei Spritzen (4, 5) über ein verzweigtes Röhrchen (6) oder separate Röhrchen (6) mit dem Behälter (3) verbunden sind, wobei die erste Spritze (4) ein Antikoagulans enthält und die zweite Spritze (5) ein Saugsystem bildet, das das Tropfen von Blut auf gravitative Weise unterstützt.

2. Kit zur Entnahme von Nabelschnurblut nach Anspruch 1, **dadurch gekennzeichnet, dass** das Entnahmeröhrchen (2) und das Röhrchen (6) Schließelemente (7) in Form von Klemmen und/oder Schließventilen aufweisen.

## Revendications

1. Kit de collecte de sang de cordon ombilical comprenant une aiguille (1), un tube de collecte (2), une première seringue (5) et un réservoir (3), **caractérisé en ce qu'**au moins un réservoir (3) est raccordé avec au moins une aiguille (1) pour la collecte de sang par le tube de collecte (2) et ledit kit comprend une seconde seringue (4), moyennant quoi les deux seringues (4, 5) sont raccordées avec le réservoir (3) par un tube ramifié (6) ou par des tubes séparés (6), dans lequel la première seringue (4) contient un anticoagulant, et la seconde seringue (5) forme un système d'aspiration, supportant le sang qui coule d'une manière gravitationnelle.

2. Kit de collecte de sang de cordon ombilical selon la revendication 1, **caractérisé en ce que** le tube de collecte (2) et le tube (6) comprennent des éléments de fermeture (7) se présentant sous la forme de clamps et/ou de valves de fermeture.
